(19) Europäisches Patentamt
European Patent Office
Office européen des brevets

(11) **EP 1 669 761 B1**

(12) **EUROPÄISCHE PATENTSCHRIFT**

(45) Veröffentlichungstag und Bekanntmachung des Hinweises auf die Patenterteilung:
**03.11.2010 Patentblatt 2010/44**

(51) Int Cl.:
*G01N 33/86* (2006.01)   *C12Q 1/56* (2006.01)

(21) Anmeldenummer: **05024888.9**

(22) Anmeldetag: **15.11.2005**

(54) **Verfahren zur automatischen Bestimmung des endogenen Thrombinpotenzials**

Method for automatically determining the endogenous thrombin potential

Procédé destiné à la détermination automatique du potentiel de thrombine endogène

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **07.12.2004 DE 102004059055**

(43) Veröffentlichungstag der Anmeldung:
**14.06.2006 Patentblatt 2006/24**

(73) Patentinhaber: **Siemens Healthcare Diagnostics Products GmbH**
**35041 Marburg (DE)**

(72) Erfinder:
• **Henckel, Thilo, Dr.**
**35083 Wetter (DE)**
• **Weyl, Andreas, Dr.**
**35117 Münchhausen (DE)**

(56) Entgegenhaltungen:
• WIELDERS S ET AL: "THE ROUTINE DETERMINATION OF THE ENDOGENOUS THROMBIN POTENTIAL, FIRST RESULTS IN DIFFERENT FORMS OF HYPER- AND HYPOCOAGULABILITY" THROMBOSIS AND HAEMOSTASIS, STUTTGART, DE, Bd. 77, Nr. 4, April 1997 (1997-04), Seiten 629-636, XP001068262 ISSN: 0340-6245
• KESSELS H ET AL: "Analysis of thrombin generation in plasma" COMPUTERS IN BIOLOGY AND MEDICINE, NEW YORK, NY, US, Bd. 24, Nr. 4, 1994, Seiten 277-288, XP002974326 ISSN: 0010-4825
• HEMKER H C ET AL: "THROMBIN GENERATION IN PLASMA: ITS ASSESSMENT VIA THE ENDOGENOUS THROMBIN POTENTIAL" THROMBOSIS AND HAEMOSTASIS, STUTTGART, DE, Bd. 74, Nr. 1, Juli 1995 (1995-07), Seiten 134-138, XP000195941 ISSN: 0340-6245
• HEMKER H C ET AL: "CONTINUOUS REGISTRATION OF THROMBIN GENERATION IN PLASMA, ITS USE FOR THE DETERMINATION OF THE THROMBIN POTENTIAL" THROMBOSIS AND HAEMOSTASIS, STUTTGART, DE, Bd. 70, Nr. 4, Januar 1993 (1993-01), Seiten 617-624, XP000567560 ISSN: 0340-6245

**Beschreibung**

Die vorliegende Erfindung betrifft ein Verfahren zur automatischen Bestimmung des endogenen Thrombinpotenzials einer Blut- oder Plasmaprobe.

Die Regulation der Hämostase erfolgt durch das Zusammenspiel verschiedener Aktivatoren, Inhibitoren sowie positiver und negativer Rückkopplungsmechanismen. Defekte in diesem Gefüge können zu einer Dysbalance im Hämostasesystem führen und entweder eine Hämorrhagie oder eine Thrombose zur Folge haben.

Thrombin, eine Serinprotease, ist das zentrale Enzym der plasmatischen Blutgerinnung, dessen Hauptfunktion die Induktion der Fibrinpolymerisation und damit der Gerinnselbildung ist. Die Bildung von Thrombin wird im Bedarfsfall durch die Aktivierung des enzymatisch inaktiven Vorläufermoleküls Prothrombin in Gang gesetzt. Um im Verletzungsfall den Gerinnungsprozess lokal und zeitlich zu begrenzen, erfolgt eine prompte Drosselung der Gerinnung, u. a. dadurch dass das freie Thrombin von inhibitorischen Faktoren wie z. B. Antithrombin oder $\alpha_2$-Makroglobulin ($\alpha_2$M) komplexiert und somit inaktiviert wird. Störungen innerhalb der Prozesse, die die Thrombinbildung oder -inhibition regulieren, können zu hyper- bzw. hypokoagulatorischen Zuständen und damit zu pathologischen Gerinnungsstörungen führen. In der Erfassung der Bildung und der Inhibition des Thrombins liegt also eine immense Aussagekraft über den jeweiligen Gerinnungszustand.

Das einer Probe innewohnende (endogene), im Falle von Plasmaproben plasmaeigene Vermögen (Potenzial) zur Bildung und Inhibition von enzymatisch aktivem, freiem Thrombin wird auch als endogenes Thrombinpotenzial (ETP) bezeichnet. Da alle biologischen Komponenten, die in einem Untersuchungsmaterial enthalten sind und die Bildung und die Inhibition von Thrombin beeinflussen können, das individuelle Thrombinpotenzial einer Probe bedingen, eignet sich die ETP-Bestimmung sowohl als globaler Test, mit dem mehrere Komponenten des Hämostasesystems erfasst werden können, als auch zur Überwachung therapeutischer Maßnahmen.

Ein Parameter, der zur Quantifizierung des endogenen Thrombinpotenzials bevorzugt bestimmt wird und der in der Literatur auch als "endogenes Thrombinpotenzial" bezeichnet wird, ist das Zeit-/Konzentrationsintegral bzw. die Fläche unter der Thrombinbildungskurve [siehe EP 0 420 332-B1, EP 0 802 986-B1 und Hemker et al. (1993) Thromb. Haemostasis 70: 617-624]. Dieser Parameter ist ein Maß für die Menge und die Aktivität des endogenen Thrombins, welches seit einer Zeit t = 0 in einer Probe von gerinnendem Blut oder Plasma anwesend war.

Zur Bestimmung des ETP wird in einer Probe gerinnungsfähigen Blutes oder Plasmas die Umsatzkinetik eines Thrombinsubstrats über die Freisetzung eines messbaren Indikators gemessen. Da die Thrombinsubstratkonzentration so eingestellt wird, dass das Substrat im Laufe der Reaktion nicht vollständig aufgebraucht werden kann, verhält sich die Menge an freigesetztem Indikator idealerweise proportional zur enzymatischen Aktivität des im Verlaufe der Gerinnungsreaktion gebildeten Thrombins.

Es ist jedoch bekannt, dass mit den derzeitig verfügbaren Thrombinsubstraten, welche eine Molekülgröße von weniger als 8 kD aufweisen, zusätzlich zur physiologisch relevanten Aktivität des freien Thrombins auch die physiologisch irrelevante Aktivität des $\alpha_2$-Makroglobulin-Thrombin-Komplexes ($\alpha_2$MT) gemessen wird. Aus der Messung der Menge an freigesetztem Indikator über die Zeit resultiert eine Reaktionskinetik, die trotz fortschreitender und schließlich vollständiger Inhibierung des freien Thrombins keine Plateauphase erreicht, sondern weiterhin ansteigt.

Um ausgehend von der gemessenen Gesamtreaktionskinetik die physiologisch relevante Thrombinkinetik T (t) herzuleiten, muss bekanntermaßen der Anteil der Aktivität des $\alpha_2$MT-Komplexes $\alpha_2$MT (t) ermittelt und von der Gesamtkinetik K (t) abgezogen werden:

$$T(t) \quad = \quad K(t) \quad - \quad \alpha_2 MT(t) \qquad (1)$$

Die Menge an $\alpha_2$MT-Komplex ist unmittelbar abhängig von der Menge an freiem Thrombin. Aus dem enzymatisch inaktiven Vorläufermolekül Prothrombin wird zunächst freies Thrombin gebildet, welches alsbald von $\alpha_2$-Makroglobulin gebunden wird, wodurch der relativ stabile $\alpha_2$MT-Komplex entsteht. Da sich also die $\alpha_2$MT-Komplex-Konzentration im Laufe der Reaktion proportional zur Thrombinkonzentration verändert, wurde der mathematische Zusammenhang zwischen der Thrombinkinetik T (t) und der Kinetik des $\alpha_2$MT-Komplexes $\alpha_2$MT (t) als Differentialgleichung beschrieben [Hemker et al. (1993) Thromb. Haemostasis 70: 617-624]:

$$\frac{\partial [\alpha_2 MT (t)]}{\partial t} = C \bullet T (t) \qquad (2)$$

mit

| a | partielles Differential |
| --- | --- |
| $\alpha_2$MT (t) | $\alpha_2$-Makroglobulin-Thrombin-Komplex-Konzentration zum Zeitpunkt t |
| T (t) | Thrombinkonzentration zum Zeitpunkt t |
| C | Konstante für die Bindung von Thrombin an $\alpha_2$M. |

Bisher bekannte Auswertungsverfahren zur Bestimmung des endogenen Thrombinpotenzials bzw. der Konzentration an freiem Thrombin [Hemker et al. (1993) Thromb. Haemostasis 70: 617-624; WO 2004/016807-A1, Wielders et al, Thromb. Haemostasis 77: 629-636; Kessels et al, Comput. Biol. Med. 24 : 277-288] beschreiben sehr komplexe Wege zur Lösung dieser Differentialgleichung (2). Die bekannten Verfahren bestimmen die Kinetik des $\alpha_2$-Makroglobulin-Thrombin-Komplexes, indem sie zunächst die 1. Ableitung der Gesamtreaktionskinetik bilden. Die infolge dieses analytischen Vorgehens erforderlichen Mittelungen können in der Summe zu Ungenauigkeiten führen. Für die Bestimmung der Konstanten C für die Bindung von Thrombin an $\alpha_2$-Makroglobulin werden komplexe Optimierungsverfahren angegeben, z. B. die modifizierte Newton Methode aus dem Software Programm "Microsoft EXCEL Solver", deren Spezifikation dem Anwender zumeist nicht bekannt ist und die auch nicht immer zu einem Ergebnis gelangt. Überdies werden eine Reihe enzymkinetischer Parameter berücksichtigt, wie z. B. die Michaelis-Konstanten spezifischer Substrate, um ausgehend von der Menge an umgesetztem Substrat auf die Menge an freiem Thrombin rückschließen zu können. Diese substratspezifischen Parameter müssen entweder aus der Literatur bekannt sein oder für das verwendete Substrat in Vorversuchen ermittelt werden.

Die Implementierung der bisher bekannten Verfahren zur Bestimmung des endogenen Thrombinpotenzials in Geräten, die im Gerinnungslabor zum Einsatz kommen, ist insofern limitiert, als dass aufgrund der Komplexität der bisher bekannten Verfahren eine hohe Datenspeicherkapazität benötigt wird und eine relativ lange Berechnungszeit resultiert. Für diagnostische Labors, die einen hohen Probendurchsatz zu bewältigen haben, sind lange Bearbeitungszeiten aus Kapazitätsgründen besonders nachteilig.

Der vorliegenden Erfindung lag also die Aufgabe zugrunde, ein vereinfachtes Verfahren zur Verfügung zu stellen, dass eine schnelle und sichere Bestimmung des endogenen Thrombinpotenzials auf üblichen Analysegeräten der Gerinnungsdiagnostik ermöglicht.

Die Lösung dieser Aufgabe besteht in der Bereitstellung der in den Ansprüchen beschriebenen erfindungsgemäßen Verfahren und Gegenständen.

Das vorliegende Verfahren zur Bestimmung des endogenen Thrombinpotenzials (ETP) einer Probe umfasst

1. die dem Fachmann bekannte Messung der Umsatzkinetik eines Thrombinsubstrates als Funktion der Zeit und
2. die erfindungsgemäße Bestimmung eines ETP-Rohwertes, der die Quantifizierung des endogenen Thrombinpotenzials einer Probe ermöglicht.

Die zur Bestimmung des endogenen Thrombinpotenzials notwendige Messung der Umsatzkinetik eines Thrombinsubstrats erfordert, dass die zu untersuchende Probe mit einem Thrombinsubstrat versetzt wird, dass die Thrombinbildung induziert wird, z. B. durch Zugabe eines Thrombinbildungsaktivators, und dass eine physikalische oder chemische Eigenschaft des umgesetzten Thrombinsubstrats als Funktion der Zeit gemessen wird.

Als Probenmaterial eignen sich z. B. Blut oder Plasma, humaner oder tierischer Herkunft. Besonders gut eignet sich plättchenarmes oder plättchenreiches Plasma, welches mit EDTA und/oder Citrat versetzt sein kann.

Als Thrombinsubstrate eignen sich z. B. Oligopeptide, die sich aus einem Teil, der eine spezifische Erkennungssequenz für Thrombin umfasst, und aus einer abgehenden Signalgruppe mit einer messbaren physikalischen Eigenschaft zusammensetzen. Bei der abgehenden Signalgruppe, die bevorzugterweise nach Abspaltung über eine veränderte physikalische Eigenschaft verfügt, kann es sich z. B. um eine chromophore, chemilumineszierende oder fluoreszente Gruppe handeln, deren Eigenschaft gemessen werden kann. Bevorzugt sind chromophore Signalgruppen deren optische Eigenschaft photometrisch bestimmt wird, wie z. B. para-Nitroanilid (pNA), dessen Absorption nach Abspaltung durch Thrombin bei einer Wellenlänge von 405 nm gemessen werden kann. Beispiele für synthetische Thrombinsubstrate, die sich zur Ausführung der vorliegenden Erfindung eignen, sind z. B. in den Ansprüchen 1 bis 5 der Patentschrift EP 0 802 986-B1 beschrieben.

Die Thrombinsubstratkonzentration ist so einzustellen, dass das Substrat im Laufe der Reaktion nicht vollständig aufgebraucht werden kann, damit sich die Menge an freigesetztem Indikator proportional zur enzymatischen Aktivität des im Verlaufe der Gerinnungsreaktion gebildeten freien und $\alpha_2$-Makroglobulin komplexierten Thrombins verhält [siehe Hemker et al. (1993) Thromb. Haemostasis 70: 617-624].

Zur Vermeidung der Entstehung störender Fibringerinnsel kann die Probe entweder mit Hilfe bekannter Verfahren zuvor defibriniert werden, wie z. B. durch die Zugabe von Schlangengiften wie beispielsweise Batroxobin, durch Hitze, durch Ausfällung oder Immunaffinitätschromatographie, oder die Probe kann mit einem Inhibitor der Fibrinpolymerisation versetzt werden. Peptide, die sich zur Hemmung der Fibrinpolymerisation eignen, ohne das in der Probe enthaltene Thrombin zu inhibieren, sind z. B. Gegenstand der Ansprüche 1 und 2 der Patentschrift EP 0 456 152-B1.

Um die Thrombinbildung zu induzieren, können z. B. Lösungen verwendet werden, die $Ca^{2+}$-Ionen und zusätzlich z. B. Thromboplastin oder einen Kontaktaktivator, wie z. B. Kaolin, Phospholipide, Schlangengift, oder Thrombomodulin und aktiviertes Protein C enthalten. Je nach diagnostischer Fragestellung kann der Fachmann aus der Vielzahl bekannter Aktivatoren der Blutgerinnung wählen, um entweder einen Teilbereich oder das gesamte Gerinnungssystem zu betrachten (siehe auch EP 0420 332-B1).

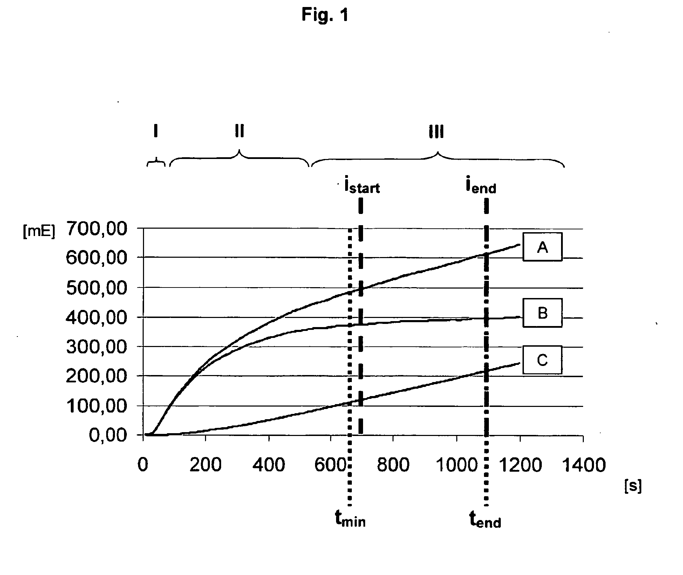

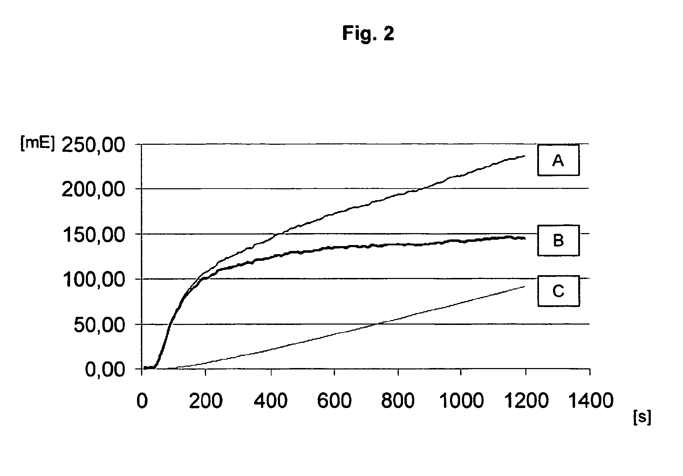

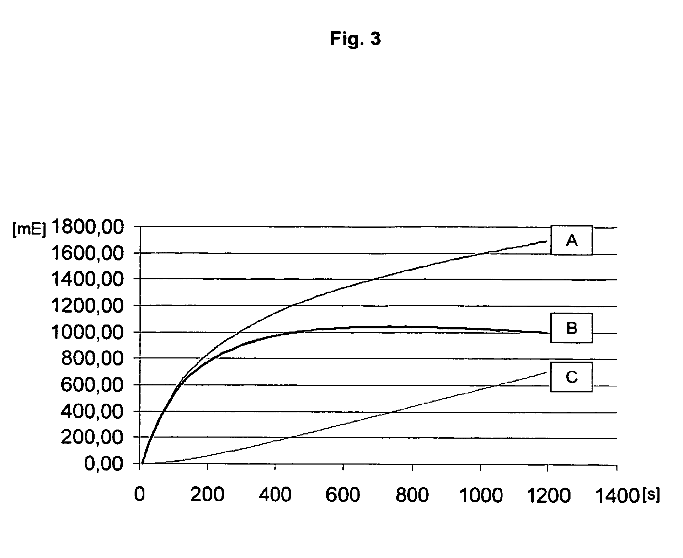

Die Messung der physikalischen Eigenschaft des umgesetzten Thrombinsubstrates erfolgt bevorzugterweise ab Zugabe des Thrombinbildungsaktivators zur Probe, vorzugsweise in Zeitintervallen von 1 bis 25 Messungen pro 10 Sekunden. Die zeitlich zugeordneten Messwerte beschreiben die Umsatzkinetik des Thrombinsubstrates bzw. die Gesamtreaktionskinetik (siehe auch Kurve A in den Figuren 1 bis 3). Die Steigung der Gesamtreaktionskurve ist ein Maß für die Reaktionsgeschwindigkeit, mit der das Thrombinsubstrat gespalten wird bzw. die Signalgruppe (Indikator) freigesetzt wird.

Die Messung der Menge an freigesetztem Indikator über die Zeit liefert eine typische Gesamtreaktionskurve, die per Sichtprüfung grob in drei Phasen eingeteilt werden kann:

a) eine erste Phase, die sogenannte Lag-Phase, die mit der Zugabe des Thrombinbildungsaktivators zum Zeitpunkt $t_0$ beginnt und durch einen flachen Kurvenverlauf, also niedrige Reaktionsgeschwindigkeit, gekennzeichnet ist. Es ist der Reaktionsabschnitt, in dem die Thrombinbildung in Gang kommt. Die Lag-Phase kann je nach Testaufbau zwischen 0 und 30 Minuten dauern.

b) eine zweite Phase, die sogenannte exponentielle Phase, die der Lag-Phase folgt und die durch einen steil ansteigenden Kurvenverlauf, also eine hohe Reaktionsgeschwindigkeit gekennzeichnet ist. Diese Phase umfasst den Reaktionsabschnitt, in dem die Thrombinbildungsrate ihr Maximum erreicht und schließlich auch die Thrombininhibitionsrate am höchsten ist. In dieser Phase erfolgt die Umsetzung des Thrombinsubstrats sowohl durch freies Thrombin wie auch durch die Aktivität des $\alpha_2$-Makroglobulin-Thrombin-Komplexes ($\alpha_2$MT). Diese Phase dauert bis zu dem Zeitpunkt, zu dem kein freies Thrombin mehr anwesend ist.

c) eine dritte Phase, die sogenannte Sättigungsphase, die durch einen kontinuierlich ansteigenden, verglichen mit der exponentiellen Phase jedoch flacheren Kurvenverlauf gekennzeichnet ist. Diese Phase umfasst den Reaktionsabschnitt, in dem die Umsetzung des Thrombinsubstrats ausschließlich auf die Aktivität des $\alpha_2$-Makroglobulin-Thrombin-Komplexes ($\alpha_2$MT) zurückzuführen ist. Diese Phase ist daran zu erkennen, dass die Reaktionsgeschwindigkeit bzw. die Menge an freigesetztem Indikator pro Zeiteinheit konstant bleibt, wodurch die Gesamtreaktionskinetik einen kontinuierlich linear ansteigenden Verlauf annimmt.

Der Verlauf der Reaktionskurve bzw. die Dauer der drei verschiedenen Reaktionsphasen hängt - neben dem endogenen Potenzial der Probe zur Bildung und Inhibition von Thrombin - von den gewählten, testspezifischen Reaktionsbedingungen ab. Beispiele für solche Reaktionsbedingungen, die der Fachmann je nach diagnostischer Fragestellung variieren kann und die sich auf die Reaktionskinetik auswirken können, sind die Art oder auch die Konzentration des verwendeten Thrombinbildungsaktivators oder die Art und Weise der Vorbehandlung des Probenmaterials. Je nach Testaufbau ist in routineartigen Vorversuchen die Gesamtreaktionsdauer empirisch zu ermitteln, um die Messdauer so anzupassen zu können, dass bei einer Messung alle drei Reaktionsphasen hinreichend berücksichtigt werden. Wie aus dem Ausführungsbeispiel und den Figuren 1 bis 3 hervorgeht, ist für die dort exemplarisch dargestellte Bestimmung des endogenen Thrombinpotenzials eine Gesamtreaktionsdauer von 20 Minuten ausreichend.

Die Messung wird so lange fortgesetzt, bis die Bildung und die Inhibition des freien Thrombins im Testansatz beendet sind und die gemessene Umsatzkinetik des Thrombinsubstrats nur noch auf die Aktivität des $\alpha_2$-Makroglobulin-Thrombin-Komplexes ($\alpha_2$MT) zurückzuführen ist. Eine besonders bevorzugte Messdauer ist dadurch gekennzeichnet, dass der Reaktionsabschnitt, in dem die Umsetzung des Thrombinsubstrats ausschließlich auf die Aktivität des $\alpha_2$-Makroglobulin-Thrombin-Komplexes ($\alpha_2$MT) zurückzuführen ist, d. h. die Sättigungsphase, einen hinreichend großen Zeitraum überspannt, so dass eine zuverlässige Bestimmung des ETP-Rohwerts möglich ist.

Das erfindungsgemäße Verfahren ist dadurch gekennzeichnet, dass zunächst innerhalb eines vorbestimmten, testspezifischen Zeitfensters der lineare Bereich der Sättigungsphase der Gesamtreaktionskinetik bestimmt wird.

Dieses Zeitfenster, dessen Anfangspunkt im folgenden auch als $t_{min}$ und dessen Endpunkt auch als $t_{end}$ bezeichnet wird, ist so festzulegen, dass es die Lag-Phase sicher ausschließt, da linear verlaufende Bereiche der Gesamtreaktionskinetik, die möglicherweise im Laufe der Lag-Phase auftreten, von der Auswertung ausgeschlossen sein sollen.

Der Anfangspunkt $t_{min}$ ist so festzulegen, dass er zeitlich hinter der Lag-Phase liegt. Bevorzugterweise wird der Anfangspunkt $t_{min}$ am Ende der exponentiellen Phase bzw. im Übergangsbereich von der exponentiellen Phase zur Sättigungsphase positioniert (siehe auch Fig. 1).

Der Endpunkt $t_{end}$ ist dann so festzulegen, dass er in der Sättigungsphase liegt und das resultierende Zeitfenster $t_{min}$ bis $t_{end}$ mindestens drei Messpunkte innerhalb der Sättigungsphase umfasst.

Bevorzugterweise wird die Position und die Größe des Zeitfensters so festgelegt, dass das Zeitfenster mehr als drei Messpunkte in der Sättigungsphase umfasst, besonders bevorzugt 4 bis 500 Messpunkte, ganz besonders bevorzugt 100 bis 300 Messpunkte.

Bei dem Zeitfenster, innerhalb welchem der lineare Bereich bestimmt werden soll, handelt es sich um einen testspezifischen Parameter, der in Abhängigkeit von den gewählten Reaktionsbedingungen festzulegen ist. Zu diesem Zweck kann der Fachmann in einer Reihe von routinemäßigen Vorversuchen den typischen Verlauf der Reaktionskurve unter gleichbleibenden Reaktionsbedingungen ermitteln. Vorteilhafterweise sind für diese Vorversuche bekannte Proben zu verwenden, die über unterschiedliche endogene Thrombinpotenziale (kurz: ETP-Werte) verfügen, damit die Variabilität der Reaktionsphasendauer bei der Vorbestimmung des testspezifischen Zeitfensters berücksichtigt werden kann. Auf diese Weise kann der Fachmann das Zeitfenster so definieren, dass bei der Auswertung unbekannter Proben sichergestellt ist, dass der Anfangspunkt $t_{min}$ zeitlich immer hinter der Lag-Phase liegt und der Endpunkt $t_{end}$ so festgelegt ist, dass das resultierende Zeitfenster mindestens drei Messpunkte der Sättigungsphase umfasst.

Die Bestimmung des linearen Bereichs innerhalb des vorbestimmten Zeitfensters erfolgt bevorzugterweise mit Hilfe eines Regressionsverfahrens.

Bei einer besonders bevorzugten Ausführungsform eines Regressionsverfahrens wird für jedes mögliche Zeitfenster, dessen Endpunkt $t_{end}$ entspricht und dessen Anfangspunkt $t_{min}$ oder einem beliebigen Messpunkt innerhalb des vorbestimmten Zeitfensters $t_{min}$ bis $t_{end}$ entspricht, das Quadrat des Pearsonschen Korrelationskoeffizienten r gebildet:

$$r = \frac{n \bullet \sum_{i=1}^{n} t_i \bullet K_i - \sum_{i=1}^{n} t_i \bullet \sum_{i=1}^{n} K_i}{\sqrt{\left[ n \bullet \sum_{i=1}^{n} t_i^2 - \left( \sum_{i=1}^{n} t_i \right)^2 \right] \bullet \left[ n \bullet \sum_{i=1}^{n} K_i^2 - \left( \sum_{i=1}^{n} K_i \right)^2 \right]}} \quad (3)$$

mit

n    Anzahl der Messpunkte im Zeitfenster
i    Zählvariable
$t_i$    Reihe mit den Zeitwerten der gemessenen Reaktionskinetik
$K_i$    Reihe mit den Messwerten der gemessenen Reaktionskinetik.

Das Quadrat des Pearsonschen Korrelationskoeffizienten $r^2$ ist ein Maß für die Linearität der Reaktionskurve in dem jeweiligen Messwertbereich bzw. Zeitfenster. $r^2$ kann Werte zwischen 0 und 1 annehmen. Je höher der Wert ist, desto besser ist die Linearität. Es wird das Maximum der Quadrate der so berechneten Pearsonschen Korrelationskoeffizienten $r_i$ bestimmt und dasjenige Zeitfenster mit dem Maximalwert, als Bereich bzw. als Reaktionsabschnitt mit dem besten linearen Verlauf ausgewählt.

Für den Fall, dass mehrere Zeitfenster den gleichen Maximalwert aufweisen, wird das größte dieser Fenster ausgewählt.

In einer weiteren bevorzugten Ausführungsform kann ein erforderlicher Mindestumfang für den linearen Bereich definiert werden. Um eine besonders zuverlässige Bestimmung des endogenen Thrombinpotenzials zu gewährleisten, mag der Fachmann festlegen, dass der lineare Bereich, der schließlich der Bestimmung des endogenen Thrombinpotenzials zugrunde gelegt werden soll, eine definierte Mindestanzahl an Messpunkten, d. h. einen definierten Mindestumfang nicht unterschreiten soll. Da der Mindestumfang des linearen Bereichs, der für die Bestimmung des ETP-Rohwerts herangezogen werden soll, abhängig gemacht werden sollte vom Verlauf der Reaktionskurve bzw. von der Streuung der Messwerte im linearen Bereich, sollte der Fachmann den Mindestumfang für die von ihm gewählten, testspezifischen Reaktionsbedingungen festlegen, so dass eine hinreichende statistische Sicherheit für die Ermittlung eines zuverlässigen ETP-Rohwerts gegeben ist. Für den Fall, dass das Zeitfenster mit dem besten linearen Verlauf den vorgegebenen Mindestumfang nicht erfüllt, kann dann zum Beispiel der Bereich für die weitergehende Auswertung verwendet werden, der genau die definierte Mindestanzahl an Messpunkten umfasst und dessen Endpunkt $t_{end}$ entspricht. Der Vorteil dieses Vorgehens besteht darin, dass z. B. auch Kinetiken, bei denen einzelne Messwerte stark abweichen ("ausreissen") nicht komplett verworfen werden müssen, sondern noch ausgewertet werden können. Ein auf solche Weise ermittelter ETP-Rohwert kann mit einem entsprechenden Hinweis ausgegeben werden, damit der Anwender zum Beispiel die Reaktionskurve per Sichtprüfung begutachten kann.

Das erfindungsgemäße Verfahren ist weiterhin dadurch gekennzeichnet, dass anhand der Steigung A der Gesamtreaktionskinetik im linearen Bereich der Sättigungsphase, die der Steigung der $\alpha_2$MT-Kinetik entspricht, die Bindungskonstante C von Thrombin an $\alpha_2$-Makroglobulin iterativ bestimmt wird.

Diesem Verfahrensschritt ist zugrunde gelegt, dass die Umsetzung des Thrombinsubstrats in der Sättigungsphase einzig auf die enzymatische Aktivität des $\alpha_2$MT-Komplexes zurückgeht. Die Reaktionsgeschwindigkeit mit der das Thrombinsubstrat gespalten wird, d. h. die Menge an freigesetztem Indikator, der pro Zeiteinheit freigesetzt wird, korreliert mit der Steigung der Gesamtreaktionskurve. Da die $\alpha_2$MT-Komplex-Konzentration in der Sättigungsphase konstant bleibt, wird pro Zeiteinheit die gleiche Menge Thrombinsubstrat gespalten, wodurch die Gesamtreaktionskurve linear ansteigt. Demzufolge kann die Steigung A des linearen Bereiches der Gesamtreaktionskinetik K (t) in der Sättigungsphase gleichgesetzt werden mit der Steigung der $\alpha_2$MT-Kinetik $\alpha_2$MT (t).

Unter Berücksichtigung der Differentialgleichung (2)

$$\frac{\partial [\alpha_2 MT\ (t)]}{\partial t} = C \bullet T(t)$$

gilt also:

$$\frac{\partial K(t)}{\partial t} = \frac{\partial [\alpha_2 MT\ (t)]}{\partial t} = C \bullet T(t) = A \qquad . \qquad (4)$$

Die Steigung A der Gesamtreaktionskinetik K (t) kann bevorzugterweise mit Hilfe einer linearen Regression berechnet werden:

$$A = \frac{n \bullet \sum t_i \bullet K_i - \left(\sum t_i\right) \bullet \left(\sum K_i\right)}{n \bullet \sum t_i^2 - \left(\sum t_i\right)^2} \qquad (5)$$

mit:

n    Anzahl der Messpunkte im linearen Bereich der Sättigungsphase
i    Zählvariable
$t_i$    Reihe mit den Zeitwerten im linearen Bereich der Sättigungsphase
$K_i$    Reihe mit den Messwerten der gemessenen Gesamtkinetik im linearen Bereich der Sättigungsphase.

Da gemäß Gleichung (4) C•T(t) gleich A ist müsste streng genommen im linearen Bereich gelten:

$$C \bullet T(t) - A = 0 \qquad . \qquad (6)$$

Da es sich jedoch um eine Reihe von fehlerbehafteten Thrombinkinetikwerten T(t$_i$) handelt, die keine ideale Gerade bilden, wird diese Forderung nicht erfüllt.

Daher ist es vorteilhaft, anstatt C•T(t$_i$) mit A gleichzusetzen, mit Hilfe eines Verfahrens zur Ausgleichsrechnung die Abweichungen der berechneten Steigung von $\alpha_2$MT, die laut Gleichung (4) gleich C•T(t$_i$) ist, von der Steigung A durch Variation von C zu minimieren. Besonders vorteilhaft ist dazu die Verwendung der Methode der kleinsten Quadrate, die auch als Methode der kleinsten Fehlerquadrate oder least squares method bezeichnet wird.

Nach der Methode der kleinsten Quadrate ist also zu fordern:

$$\sum_{i=istart}^{iend} (C \bullet T_i - A)^2 = Min! \tag{7}$$

mit

$T_i = T(t_i)$     Thrombinkinetikwert zum Zeitpunkt $t_i$

istart     Index des Beginns des linearen Bereiches

iend     Index des Endes des linearen Bereiches.

Notwendige Bedingung zur Erfüllung der Forderung ist, dass die Ableitung nach C zu 0 wird. C ist also so zu bestimmen, dass

$$\frac{\partial \sum_{i=istart}^{iend} (C \bullet T_i - A)^2}{\partial C} = 0 \tag{8}$$

Durch Auflösen der Gleichung nach C erhält man:

$$C = A \bullet \frac{\sum_{i=istart}^{iend} T_i}{\sum_{i=istart}^{iend} T_i^2} \tag{9}$$

Da es für die Konstante C eine Rückkopplung mit der Gleichung (2) gibt, weil der Wert der Konstanten C abhängt von der Thrombinkinetik T, die wegen Gleichung (13) wiederum eine Funktion der Konstanten C ist, wird C iterativ bestimmt, indem die Ergebnisse eines Iterationsschrittes als Ausgangswerte des jeweils nächsten Iterationsschrittes genommen werden:

$$C_{j+1} = A \bullet \frac{\sum T_i(C_j)}{\sum T_i^2(C_j)} \qquad (10)$$

mit $C_0 = 0$
und

i    Zählindex für die Thrombinkinetikwerte $T(t_i)$
j    Zählindex für die Iterationen.

Zur Beendung der Iteration wird ein Abbruchkriterium definiert. Das Abbruchkriterium ist vom Fachmann testspezifisch, in Abhängigkeit von den gewählten Reaktionsbedingungen in einer Reihe von routinemäßigen Vorversuchen festzulegen, indem geprüft wird, ab welchem Zeitpunkt die berechnete Thrombinkinetik sich nicht mehr signifikant verändert. Bevorzugterweise wird das Abbruchkriterium so definiert, dass, wenn die Abweichung $|C_j - C_{j+1}|$ größer als ein vorbestimmter Maximalwert $\varepsilon$ ist, $C_j = C_{j+1}$ gesetzt wird und Gleichung 13 mit dem neuen $C_j$ erneut berechnet wird, bis die Abweichung $|C_j - C_{j+1}|$ $\varepsilon$ nicht mehr überschreitet.

Das vorliegende Verfahren ist weiterhin dadurch gekennzeichnet, dass die Werte der $\alpha_2$MT-Kinetik $\alpha_2$MT (t) ermittelt werden. Mit Hilfe der ermittelten Bindungskonstanten C kann nun für jeden Zeitpunkt der $\alpha_2$MT-Kinetikwert $\alpha_2$MT (t) ermittelt werden. Dafür wird die Differentialgleichung (2) unter Berücksichtigung von Gleichung (1) numerisch gelöst und es folgt:

$$\frac{\partial\left[\alpha_2\mathrm{MT}(t)\right]}{\partial t} + C \bullet \alpha_2\mathrm{MT}(t) = C \bullet K(t) \qquad . \qquad (11)$$

Gleichung (11) wird dann als finite Differenz formuliert [dazu siehe auch Fritsch, Herbert: Finite-Differenzen-Methode. IRB Verlag Stuttgart, 1998 (ISBN 3816712797) oder Marsal, Dietrich: Finite Differenzen und Elemente. Numerische Lösung von Variationsproblemen und partiellen Differentialgleichungen. Springer-Verlag Berlin Heidelberg, 1989 (ISBN 3540501924)]:

$$\frac{\alpha_2\mathrm{MT}_{i+1} - \alpha_2\mathrm{MT}_i}{t_{i+1} - t_i} + C \bullet \alpha_2\mathrm{MT}_i = C \bullet K_i \qquad , \qquad (12)$$

woraus dann die Zeitreihe für die $\alpha_2$MT-Kinetikwerte entwickelt werden kann:

$$\alpha_2\mathrm{MT}_{i+1} = C \bullet K_i \bullet (t_{i+1} - t_i) - \alpha_2\mathrm{MT}_i \bullet (C \bullet (t_{i+1} - t_i) - 1) \qquad (13)$$

mit $\alpha_2$MT$_0$=0.

Mit Hilfe der in Gleichung (10) berechneten Bindungskonstanten C, den gemessenen Kinetikwerten K und den dazugehörigen Zeitwerten t kann mit Hilfe von Gleichung (13) der $\alpha_2$MT-Kinetikwert $\alpha_2$MT (t) für jeden Zeitpunkt ermittelt werden.

Das erfindungsgemäße Verfahren ist weiterhin dadurch gekennzeichnet, dass die Werte der $\alpha_2$MT-Kinetik von den dazugehörigen Werten der Gesamtreaktionskinetik abgezogen werden. Zur Bestimmung des Thrombinkinetikwertes T (t) zu dem Zeitpunkt i+1 wird die Differenz zwischen dem zum Zeitpunkt i+1 gemessenem Gesamtreaktionskinetikwert K (t) und dem für den vorangegangenen Zeitpunkt i ermittelten $\alpha_2$MT-Kinetikwert $\alpha_2$MT (t) gebildet:

$$T(t_{i+1}) = K(t_{i+1}) - \alpha_2 MT(t_i) \qquad . \tag{14}$$

Das erfindungsgemäße Verfahren ist weiterhin dadurch gekennzeichnet, dass von den Werten, die für die Kinetik des freien Thrombins T(t) im linearen Bereich der Sättigungsphase ermittelt wurden, der Mittelwert gebildet wird.

Im linearen Bereich der Sättigungsphase der Gesamtreaktionskinetik sollte die Thrombinkinetik T(t) nahezu parallel zur Zeitachse verlaufen, die berechneten Thrombinwerte also annähernd konstant sein. Der Mittelwert der Werte der Kinetik des freien Thrombins T(t), die für den linearen Bereich der Sättigungsphase der Gesamtreaktionskinetik (istart bis iend) bestimmt wurden, kann auch als ETP-Rohwert (Etpr) bezeichnet werden:

$$Etpr = \frac{\sum_{i=istart}^{iend} T_i}{n} \qquad . \tag{15}$$

In einer bevorzugten Ausführungsform kann mit Hilfe von statistischen Verfahren die Güte des ermittelten ETP-Rohwerts überprüft werden, bevorzugterweise durch die Bildung des Variationskoeffizienten $V_k$ der Kinetikwerte des freien Thrombins die zur Mittelung herangezogen wurden:

$$V_k = \frac{\sqrt{\dfrac{n \bullet \sum\limits_{i=istart}^{iend} T_i^2 - \left(\sum\limits_{i=istart}^{iend} T_i\right)^2}{n \bullet (n-1)}}}{Etpr} \bullet \quad 100\% \qquad . \tag{16}$$

Der mit Hilfe des erfindungsgemäßen Verfahrens ermittelte ETP-Rohwert einer Probe hat die Einheit der gemessenen physikalischen Eigenschaft des umgesetzten Thrombinsubstrates, wie z. B. optische Dichte, Absorption in [mE] oder dergleichen.

In einer bevorzugten Ausführungsform kann der Gerinnungsstatus eines Patienten bzw. einer Patientenprobe durch den Vergleich mit dem ETP-Rohwert einer Normalstandardprobe festgestellt werden. Als Normalstandardproben eignen sich beispielsweise Pools von Blut- oder Plasmaproben gesunder Probanden. ETP-Rohwerte, die oberhalb eines Normalstandard-ETP-Rohwertes liegen, weisen auf eine thrombophile Disposition hin. ETP-Rohwerte, die unterhalb eines Normalstandard-ETP-Rohwertes liegen, weisen auf eine hämorrhagische Disposition hin. Des weiteren ermöglicht der Vergleich des ETP-Rohwertes einer Probe mit den ETP-Rohwerten von Proben bekannter Thrombin- bzw. Prothrombinkonzentration eine Quantifizierung des in der Probe gebildeten Thrombins.

Ein weiterer Gegenstand der vorliegenden Erfindung betrifft Geräte, die dadurch gekennzeichnet sind, dass das erfindungsgemäße Verfahren zur Bestimmung des endogenen Thrombinpotenzials implementiert ist, und die auf den Befehl einer Bedienperson die aufgezeichneten Messdaten mit Hilfe des erfindungsgemäßen Verfahrens automatisch auswerten können. Dabei kann es sich um Instrumente wie z. B. automatische Gerinnungsanalyzer handeln, die neben einer Einheit für die elektronische Datenverarbeitung auch über Vorrichtungen zur Manipulation von Proben und Reagenzien und/oder Vorrichtungen zum Messen physikalischer Eigenschaften der Proben oder Testansätze verfügen können.

Ein weiterer Gegenstand der vorliegenden Erfindung betrifft Speichermedien, auf welchen das erfindungsgemäße Verfahren in Form eines Computerprogramms abgespeichert ist. Dazu gehören neben Festplatten auch Wechseldatenträger, wie z. B. Disketten, CDs oder DVDs.

Das im folgenden beschriebene Ausführungsbeispiel dient der Veranschaulichung des erfindungsgemäßen Verfahrens und ist nicht als Einschränkung zu verstehen.

**Beispiel**

**Automatische Bestimmung des ETP-Rohwertes von Plasmaproben**

Zur Bestimmung des ETP-Rohwerts von humanen Plasmaproben wurde ein chromogenes Testverfahren angewendet, wie es in EP 0 420 332-B1 beschrieben ist.

135 $\mu$l plättchenarmes Plasma (PPP) wurden mit 40 $\mu$l Puffer (50 mM Tris-HCl, pH 7,4) und mit 40 $\mu$l einer Lösung vermischt, die ein para-Nitroanilid (pNA)-gekoppeltes Oligopeptid, das spezifisch von Thrombin gespalten wird (Pefachrome®TG; Pentapharm LTD, Schweiz) und einen Inhibitor der Fibrinpolymerisation (Peptidamid H-Gly-Pro-Arg-Pro-Ala-NH$_2$; siehe EP 0 456 152-B1) enthielt. Nach einer 7minütigen Inkubation wurden dem Ansatz 15 $\mu$l CaCl$_2$ (250 mM) und 30 $\mu$l Innovin® (Reagenz bestehend aus rekombinantem, humanem Gewebefaktor und einer Mischung von synthetischen Phospholipiden; Dade Behring Marburg GmbH, Deutschland) als Thrombinbildungaktivator zugegeben und mit der Messung der Absorption bei einer Wellenlänge von 405 nm begonnen. Die Messung der Absorption erfolgte in einem Zeitintervall von 1 bis 2 Messungen pro Sekunde und über einen Zeitraum von 20 Minuten. Die Messwerte wurden in Abhängigkeit von der Zeit kontinuierlich aufgezeichnet. Anhand der so bestimmten Gesamtreaktionskinetik wurde anschließend mit Hilfe des erfindungsgemäßen Verfahrens der ETP-Rohwert der Probe ermittelt. Für die gewählten testspezifischen Bedingungen war zur Bestimmung des linearen Bereichs der Sättigungsphase der Gesamtreaktionskinetik ein Zeitfenster mit dem Anfangspunkt $t_{min}$ = 650 Sekunden und dem Endpunkt $t_{end}$ = 1078 Sekunden vorbestimmt worden.

Das Mischen der Proben mit den Reagenzien, die Messung der Absorption und die automatische Bestimmung des ETP-Rohwertes erfolgten vollautomatisch auf einem BCS® Gerinnungsanalyzer (Dade Behring Marburg GmbH, Deutschland), auf welchem das erfindungsgemäße Verfahren in Form einer Software implementiert war.

Auf diese Weise wurden eine Normalstandardprobe (Fig. 1) und zwei pathologische Patientenproben (Fig. 2 und 3) untersucht.

**Figuren**

In den Figuren 1, 2 und 3 sind jeweils die gemessene Gesamtreaktionskinetik K(t) (Kurve A), d. h. die gemessenen Absorptions-Werte [mE] in Abhängigkeit von der Zeit [s], und die mit Hilfe des erfindungsgemäßen Verfahrens ermittelten Kinetiken des $\alpha_2$MT-Makroglobulin-Thrombin-Komplexes $\alpha_2$MT(t) (Kurve C) sowie des freien Thrombins T(t) (Kurve B) graphisch dargestellt.

Figur 1

Graphische Darstellung der gemessenen Gesamtreaktionskinetik (A) einer Normalplasmaprobe und der gemäß dem erfindungsgemäßen Verfahren ermittelten $\alpha_2$MT- und Thrombinkinetik. Zur Veranschaulichung wurde die ungefähre Dauer der drei Reaktionsphasen der Gesamtreaktionskurve (A) eingezeichnet, wobei I = lag-Phase, II = exponentielle Phase und III = Sättigungsphase bedeutet. Ausserdem wurde das hier verwendete, testspezifische Zeitfenster $t_{min}$ = 650 s bis $t_{end}$ = 1078 s eingezeichnet (gepunktete Linien), innerhalb welchem nach dem Bereich der Gesamtreaktionskinetik K (t) mit der besten Linearität gesucht wurde. Der lineare Bereich (gestrichelte Linien) der Gesamtreaktionskinetik wurde zwischen den Punkten $i_{start}$ = 672 s und $i_{end}$ = $t_{end}$ = 1078 s ermittelt. Innerhalb dieses linearen Bereiches verläuft die Kurve der ermittelten Thrombinkinetik (B) nahezu parallel zur X-Achse. Durch Mittelung der Thrombinwerte, die innerhalb dieses Zeitfensters des linearen Bereichs ermittelt wurden, wurde ein ETP-Rohwert von 378 mE mit einem Variationskoeffizienten der Rohwertermittlung von 2,9 % bestimmt.

Figur 2

Graphische Darstellung der gemessenen Gesamtreaktionskinetik einer Plasmaprobe eines unter oraler Antikoagulation stehenden Patienten und der gemäß dem erfindungsgemäßen Verfahren ermittelten $\alpha_2$MT- und Thrombinkinetik. Durch Mittelung der Werte der Kinetik des freien Thrombins, die für den linearen Bereich der Gesamtreaktionskinetik bestimmt wurden, wurde ein ETP-Rohwert von 136 mE bestimmt, der gegenüber dem ETP-Rohwert der Normalplasmaprobe signifikant erniedrigt ist und einen hämorrhagischen Gerinnungsstatus des Patienten indiziert. Der Variationskoeffizient der Rohwertermittlung beträgt 2,3 %.

Figur 3

Graphische Darstellung der gemessenen Gesamtreaktionskinetik einer pathologischen Plasmaprobe und der gemäß dem erfindungsgemäßen Verfahren ermittelten $\alpha_2$MT- und Thrombinkinetik. Durch Mittelung der Werte der Kinetik des freien Thrombins, die für den linearen Bereich der Gesamtreaktionskinetik bestimmt wurden, wurde ein ETP-Rohwert von 1034 mE bestimmt, der gegenüber dem ETP-Rohwert der Normalplasmaprobe signifikant erhöht ist und einen thrombophilen Gerinnungsstatus des Patienten indiziert. Der Variationskoeffizient der Rohwertermittlung beträgt 0,7 %.

**Patentansprüche**

1. Verfahren zur Bestimmung des endogenen Thrombinpotenzials (ETP) einer Probe von gerinnendem Blut oder Plasma, wobei die Umsatzkinetik eines Thrombinsubstrates als Funktion der Zeit gemessen wird,
**dadurch gekennzeichnet dass**

a) innerhalb eines vorbestimmten, testspezifischen Zeitfensters der lineare Bereich der Sättigungsphase der Gesamtreaktionskinetik bestimmt wird, und dann
b) anhand der Steigung A der Gesamtreaktionskinetik im linearen Bereich der Sättigungsphase, die der Steigung der $\alpha_2$MT-Kinetik entspricht, die Bindungskonstante C von Thrombin an $\alpha_2$-Makrogiobulin gemäß folgender Gleichung iterativ bestimmt wird:

$$C_{j+1} = A \bullet \frac{\sum T_i(C_j)}{\sum T_i^2(C_j)} \qquad (10)$$

mit $C_0 = 0$
und

i Zählindex für die Thrombinkinetikwerte T zum Zeitpunkt $t_i$,
j Zählindex für die Iterationen,

und dann
c) die Werte der $\alpha_2$MT-Kinetik ermittelt und von den dazugehörigen Werten der Gesamtreaktionskinetik abgezogen werden, und dann
d) durch Mittelung der Werte der Kinetik des freien Thrombins, die für den linearen Bereich der Gesamtreaktionskinetik in der Sättigungsphase bestimmt wurden, der ETP-Rohwert ermittelt wird.

2. Verfahren nach Anspruch 1 **dadurch gekennzeichnet, dass** das testspezifische Zeitfenster mindestens 3 Messpunkte innerhalb der Sättigungsphase umfasst.

3. Verfahren nach Anspruch 1 und 2 **dadurch gekennzeichnet, dass** der lineare Bereich der Sättigungsphase der gemessenen Gesamtreaktionskinetik mit Hilfe eines Regressionsverfahrens ermittelt wird.

4. Verfahren nach Anspruch 3 **dadurch gekennzeichnet, dass** für jedes mögliche Zeitfenster, dessen Endpunkt dem Endpunkt des vorbestimmten, testspezifischen Zeitfensters entspricht und dessen Anfangspunkt dem Anfangspunkt des vorbestimmten, testspezifischen Zeitfensters oder einem beliebigen Messpunkt innerhalb des vorbestimmten Zeitfensters entspricht, das Quadrat des Pearsonschen Korrelationskoeffizienten r gebildet wird.

5. Verfahren nach Anspruch 4 **dadurch gekennzeichnet, dass** das Maximum der Quadrate der Korrelationskoeffizienten $r_i$ bestimmt wird und dasjenige Zeitfenster mit dem Maximalwert als linearer Bereich der Gesamtreaktionskinetik gewählt wird.

6. Verfahren nach Anspruch 5 **dadurch gekennzeichnet, dass** für den Fall, dass der bestimmte lineare Bereich eine definierte Minimalbreite unterschreitet, ein Fenster einer zuvor definierten Mindestbreite als linearer Bereich gewählt wird.

7. Verfahren nach Anspruch 1 **dadurch gekennzeichnet, dass** die Steigung A der Gesamtreaktionskinetik im linearen Bereich der Sättigungsphase mit Hilfe einer linearen Regression berechnet wird.

8. Verfahren Anspruch 7 **dadurch gekennzeichnet, dass** mit Hilfe eines Verfahrens zur Ausgleichsrechnung die Abweichungen der berechneten Steigung von $\alpha_2$MT von der Steigung A der Gesamtreaktionskinetik durch Variation der Bindungskonstanten C von Thrombin an $\alpha_2$M minimiert werden.

9. Verfahren nach Anspruch 8 **dadurch gekennzeichnet, dass** die Methode der kleinsten Quadrate als Verfahren zur Ausgleichsrechnung verwendet wird.

10. Verfahren nach Anspruch 1 **dadurch gekennzeichnet, dass** die Berechnung der Zeitreihe der $\alpha_2$MT-Kinetikwerte unter Anwendung des Verfahrens der finiten Differenzen durchgeführt wird.

11. Verfahren nach einem der Ansprüche 1 bis 10 **dadurch gekennzeichnet, dass** mit dem Variationskoeffizienten der Werte der Kinetik des freien Thrombins im linearen Bereich eine Gütekontrolle des ETP-Rohwertes erfolgt.

12. Verfahren nach einem der Ansprüche 1 bis 11 **dadurch gekennzeichnet, dass** der Gerinnungsstatus eines Patienten bzw. einer Patientenprobe durch den Vergleich mit dem ETP-Rohwert einer Normalstandardprobe festgestellt wird.

13. Zur Bestimmung des endogenen Thrombinpotenzials geeignetes Gerät **dadurch gekennzeichnet, dass** ein Bestimmungsverfahren gemäß einem der Ansprüche 1 bis 12 darin implementiert ist.

**Claims**

1. Method for determining the endogenous thrombin potential (ETP) of a sample of coagulating blood or plasma, with the turnover kinetics of a thrombin substrate being measured as a function of time,
   **characterized in that**

   a) the linear region of the saturation phase of the overall reaction kinetics is determined within a predetermined, test-specific time window, and then
   b) the binding constant C of thrombin to $\alpha_2$-macroglobulin is determined iteratively according to the following equation:

$$C_{j+1} = A \bullet \frac{\sum T_i(C_j)}{\sum T_i^2(C_j)} \qquad (10)$$

   in which $C_0 = 0$
   and

   i is the numeric index for the thrombin kinetics values T at the time $t_i$, and
   j is the numeric index for the iterations, using the slope A of the overall reaction kinetics in the linear region of the saturation

   phase, which slope corresponds to the slope of the $\alpha_2$MT kinetics, and then
   c) the values of the $\alpha_2$MT kinetics are determined and subtracted from the appurtenant values of the overall reaction kinetics, and then

d) the raw ETP value is determined by averaging the values, which were determined for the linear region of the overall reaction kinetics in the saturation phase, of the kinetics of the free thrombin.

2. Method according to Claim 1, **characterized in that** the test-specific time window encompasses at least 3 measurement points within the saturation phase.

3. Method according to Claims 1 and 2, **characterized in that** the linear region of the saturation phase of the measured overall reaction kinetics is determined using a regression method.

4. Method according to Claim 3, **characterized in that** the square of the Pearson's correlation coefficient r is formed for each possible time window whose end point corresponds to the end point of the predetermined, test-specific time window and whose starting point corresponds to the starting point of the predetermined, test-specific time window or an arbitrary measurement point within the predetermined time window.

5. Method according to Claim 4, **characterized in that** the maximum of the squares of the correlation coefficients $r_i$ is determined and the time window having the maximum value is selected as the linear region of the overall reaction kinetics.

6. Method according to Claim 5, **characterized in that**, if the determined linear region falls below a defined minimum width, a window of a previously defined minimum width is selected as the linear region.

7. Method according to Claim 1, **characterized in that** the slope A of the overall reaction kinetics in the linear region of the saturation phase is calculated using a linear regression.

8. Method according to Claim 7, **characterized in that** a method of compensation calculation is used to minimize the deviations of the calculated slope of $\alpha_2$MT from the slope A of the overall reaction kinetics due to variation of the binding constants C of thrombin to $\alpha_2$M.

9. Method according to Claim 8, **characterized in that** the method of least squares is used as the method of compensation calculation.

10. Method according to Claim 1, **characterized in that** the time series of the $\alpha_2$MT kinetic values is calculated using the method of finite differences.

11. Method according to one of Claims 1 to 10, **characterized in that** the coefficient of variation of the values of the kinetics of the free thrombin in the linear region is used to effect a quality control of the raw ETP value.

12. Method according to one of Claims 1 to 11, **characterized in that** the coagulation status of a patient or of a patient sample is established by comparing with the raw ETP value of a normal standard sample.

13. Instrument which is suitable for determining the endogenous thrombin potential, **characterized in that** a determination method according to one of Claims 1 to 12 is implemented therein.

**Revendications**

1. Procédé de détermination du potentiel de thrombine endogène ( PTE ) d'un échantillon de sang se coagulant ou de plasma, en mesurant la cinétique de conversion d'un substrat de thrombine en fonction du temps,
   **caractérisé en ce que**

   a) dans un créneau temporel spécifique au test et déterminé à l'avance, on détermine le domaine linéaire de la phase de saturation de la cinétique globale de réaction, et ensuite
   b) au moyen de la pente A de la cinétique globale de réaction dans le domaine linéaire de la phase de saturation, qui correspond à la pente de la cinétique $\alpha_2$MT, on détermine par itération la constante C de fixation de thrombine à une macroglobuline $\alpha_2$ selon l'équation suivante

$$C_{j+1} = A \bullet \frac{\sum \dot{T}_i(C_j)}{\sum T_i^2(C_j)} \qquad (10)$$

avec $C_0 = 0$
et

i indice de viscosité pour les valeurs T de cinétique de thrombine à l'instant $t_i$
j indice de viscosité pour les itérations

et ensuite
c) on détermine les valeurs de la cinétique $\alpha_2$MT et à partir des valeurs associées on déduit la cinétique globale de réaction, et ensuite
d) en faisant la moyenne des valeurs de la cinétique de la thrombine libre, qui ont été déterminés pour le domaine linéaire de la cinétique globale de réaction de la phase de saturation, on détermine la valeur brute de PTE.

2. Procédé suivant la revendication 1, **caractérisé en ce que** le créneau temporel spécifique au test comprend au moins trois points de mesure dans la phase de saturation.

3. Procédé suivant la revendication 1 et 2, **caractérisé en ce que** l'on détermine le domaine linéaire de la phase de saturation de la cinétique globale de réaction mesurée à l'aide d'un procédé de régression.

4. Procédé suivant la revendication 3, **caractérisé en ce que** pour chaque créneau temporel possible, dont la fin correspond à la fin du créneau temporel spécifique au test déterminé à l'avance et dont le début correspond au début du créneau temporel spécifique au test déterminé à l'avance ou à un point de mesure quelconque dans le créneau temporel déterminé à l'avance, on forme le carré du coefficient r de corrélation de Pearson.

5. Procédé suivant la revendication 4, **caractérisé en ce qu'** on détermine le maximum du carré des coefficients $r_i$ de corrélation et on choisit le créneau temporel ayant la valeur maximum comme domaine linéaire de la cinétique globale de réaction.

6. Procédé suivant la revendication 5, **caractérisé en ce que**, dans le cas où le domaine linéaire déterminé est inférieur à une largeur minimum définie, on choisit comme domaine linéaire un créneau d'une largeur minimum définie auparavant.

7. Procédé suivant la revendication 1, **caractérisé en ce que** l'on calcule la pente A de la cinétique globale de réaction dans le domaine linéaire de la phase de saturation à l'aide d'une régression linéaire.

8. Procédé suivant la revendication 7, **caractérisé en ce que**, à l'aide d'un procédé de calcul de compensation, on minimise les écarts de la pente calculée de $\alpha_2$MT à la pente A de la cinétique globale de réaction, en faisant varier la constante C de fixation de la thrombine $\alpha_2$M.

9. Procédé suivant la revendication 8, **caractérisé en ce qu'** on utilise la méthode des moindres carré comme procédé de calcul de compensation.

10. Procédé suivant la revendication 1, **caractérisé en ce que** l'on effectue le calcul de la série temporelle des valeurs cinétiques $\alpha_2$MT en utilisant le procédé des différences finies.

11. Procédé suivant l'une des revendications 1 à 10, **caractérisé en ce que**, par le coefficient de variation des valeurs de la cinétique de la thrombine libre dans le domaine linéaire, on effectue un contrôle de qualité de la valeur brute de PTE.

12. Procédé suivant l'une des revendications 1 à 11, **caractérisé en ce que** l'on constate l'état de coagulation d'un patient ou d'un échantillon de patient en comparant la valeur brute de PTE à un échantillon standard normal.

13. Appareil qui convient pour la détermination du potentiel de thrombine endogène, **caractérisé en ce que** l'on y met en oeuvre un procédé de détermination suivant l'une des revendications 1 à 12.

## Fig. 1

## Fig. 2

**Fig. 3**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 0420332 B1
- EP 0802986 B1
- WO 2004016807 A1
- EP 0456152 B1

**In der Beschreibung aufgeführte Nicht-Patentliteratur**

- **Hemker et al.** *Thromb. Haemostasis,* 1993, vol. 70, 617-624
- **Wielders et al.** *Thromb. Haemostasis,* vol. 77, 629-636
- **Kessels et al.** *Comput. Biol. Med.,* vol. 24, 277-288
- **Fritsch, Herbert.** Finite-Differenzen-Methode. IRB Verlag Stuttgart, 1998
- **Marsal, Dietrich.** Finite Differenzen und Elemente. Numerische Lösung von Variationsproblemen und partiellen Differentialgleichungen. Springer-Verlag Berlin Heidelberg, 1989